# EUROPÄISCHE PATENTSCHRIFT

(11) **EP 2 043 995 B1**
(45) Veröffentlichungstag und Bekanntmachung des Hinweises auf die Patenterteilung: **02.01.2013**
(21) Anmeldenummer: 07786010.4
(22) Anmeldetag: 12.07.2007
(51) Int. Cl.: C07C 205/09, C07C 205/19, C07C 205/26, C07C 205/34, C07C 201/12, C07C 205/13

(54) **VERFAHREN ZUM HERSTELLEN VON IN 1'-STELLUNG UNVERZWEIGTEN ALKENYLNITROBENZOL-DERIVATEN**
A METHOD FOR PRODUCING ALKENYL NITROBENZOL DERIVATIVES UNBRANCHED IN THE 1-POSITION
PROCEDE POUR LA PREPARATION DE DERIVES ALCENYLNITROBENZENE NON RAMIFIES EN POSITION 1'

(30) Priorität: 14.07.2006 DE 102006033092
(43) Veröffentlichungstag der Anmeldung: 08.04.2009
(73) Patentinhaber: Bayer CropScience AG, 40789 Monheim (DE)
(72) Erfinder: STRAUB, Alexander, 42117 Wuppertal (DE); GREUL, Jörg, Nico, 42799 Leichlingen (DE); LUI, Norbert, 51519 Odenthal (DE)
(86) Internationale Anmeldenummer: PCT/EP2007/006178
(87) Internationale Veröffentlichungsnummer: WO 2008/006576

(56) Entgegenhaltungen:
- WO-A-03/074491
- WO-A-2006/087221
- SHIGERU MURATA ET.AL.: "Mechanistic studies of intramolecular CH insertion reaction of arylnitrenes: isotope effect, configurational purity and radical clock studies" J. OF PHYSICAL ORGANIC CHEM., Bd. 18, 2005, Seiten 9-20, XP002455705

## Beschreibung

Die vorliegende Erfindung betrifft ein Verfahren zum Herstellen von Alkenylnitrobenzol-Derivaten, die als Zwischenprodukte für fungizid wirksame Alkylaniliden Bedeutung haben.

Im Stand der Technik sind bereits Herstellungsmethoden für in 1'-Position unverzweigte Alkylaniline beschrieben. Genannt sei die Friedel-Crafts-Acylierung von Anilinen mit Säurechloriden und anschließender Reduktion der entstandenen Ketone (EP-A-824099) oder die Palladium-, bzw. Kupfer-katalysierte Umsetzung von Bromalkylbenzolen mit Benzophenonimin, bzw. Ammoniak, ggfs. gefolgt von der Abspaltung der Schutzgruppe mit Hydroxylamin (WO-A-03074491 und WO-A-06061226).

Alkylnitrobenzole können durch Reduktion der Nitrogruppe in Alkylaniline überführt werden und wurden bisher z.B. durch die Nitrierung von Alkylaromaten, wie in EP-A-824099 und WO-A-03074491 beschrieben oder durch Reaktion von Nitrobenzolderivaten mit Grignard-Reagenzien, beschrieben in J. Org. Chem. 1980, 45, 522, erhalten.

Nitrogruppen können jedoch in Gegenwart von Grignard-Reaktionen vielfältige Redox-Nebenprodukte ergeben.

Die JP-A-62-202091 beschreibt die Umsetzung von Isobutyraldehyd mit 2-Nitrotoluol, wobei lediglich 2,2 % 2-(2-Nitrophenyl)-1-isopropylethanol erhalten wurden.

In J. Organomet. Chem. (2006), 691(8), 1462 wird die Synthese von 1-[3,3-Dimethylbut-1-en-1-yl]-2-nitrobenzol ausgehend von 2 Nitrobenzoylchlorid beschrieben. Aufgrund der hohen Kosten und der Toxizität der dort verwendeten Reagenzien, z.B. Me₃SnF, Polymethylhydroxysiloxan und Pd₂(dba)₃, kann die Methode nicht wirtschaftlich in einem industriellen Verfahren praktiziert werden.

Folglich sind die im Stand der Technik beschriebenen Verfahren entweder zu unselektiv, zu komplex und/oder zu kostenintensiv.

Der Erfindung liegt somit die Aufgabe zugrunde, ein Verfahren zum Herstellen von Alkylnitrobenzolen zur Verfügung zu stellen, welches die mit dem Stand der Technik verbundenen Nachteile nicht aufweist.

Überraschenderweise wurde nun gefunden, dass die Umsetzung von 2-Nitrotoluol mit Aldehyden wie Pivalaldehyd oder Cyclopropylaldehyd in guten Ausbeuten erfolgt.

Gegenstand der vorliegenden Erfindung ist daher ein Verfahren zum Herstellen von Alkenylnitrobenzolen der Formel (I) 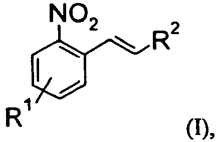 wobei
- R¹: für Wasserstoff, Halogen, -CR'(CF₃)₂ steht, wobei R' ausgewählt ist aus H, F oder O-C₁₋₄-Alkyl und besonders bevorzugt Wasserstoff ist
- R²: für Cyclopropyl oder t-Butyl steht,
umfassend die Schritte
(i) Umsetzen von 2-Nitrotoluolen der Formel (II), 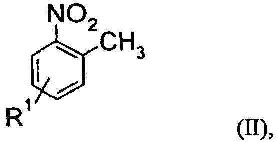
   worin R¹ die obige Bedeutung hat, mit Aldehyden der Formel (III),

      R²-CHO (III),
   worin R² die obige Bedeutung hat, zu Hydroxalkylnitrobenzolen der Formel (IV);
(ii) Umsetzen der aus Schritt (i) erhaltenen Hydroxyalkylnitrobenzole der Formel (IV) 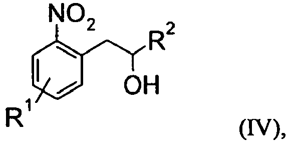
   worin R¹ und R² die obigen Bedeutungen haben, zu Halogenalkylnitrobenzolen der Formel (V) 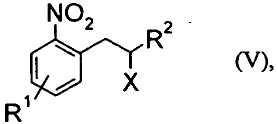
   worin R¹ und R² die obigen Bedeutungen haben und
   X ein Halogenatom oder eine Abgangsgruppe wie Tosylat (OTos), Mesylat (OMes) oder OSOCI ist,
   und
(iii) anschließende Eliminierung der Alkylnitrobenzole der Formel (V) zu Alkenylnitrobenzolen der Formel (I)
oder
(iv) direkte Eliminierung der Hydroxyalkylnitrobenzole der Formel (IV) zu Alkenylnitrobenzolen der Formel (I).

Weitere Gegenstände der vorliegenden Erfindung sind die Zwischenprodukte der Formel (I) 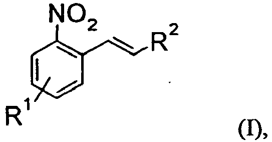 wobei
- R¹: für Halogen oder -CR'(CF₃)₂ steht und R' ausgewählt ist aus H, F oder O-C₁₋₄-Alkyl und besonders bevorzugt für Wasserstoff ist und
- R²: für t-Butyl oder Cyclopropyl steht und
sich der Substituent R¹ vorzugsweise in meta- oder para-Position, besonders bevorzugt in 4-Position (para zur NO₂-Gruppe) des Aromaten befindet;
der Formel (IV) 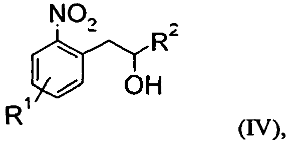 wobei
- R¹: für Wasserstoff, Halogen, -CR'(CF₃)₂ steht und R' ausgewählt ist aus H, F oder O-C₁₋₄-Alkyl und besonders bevorzugt Wasserstoff ist und
- R²: für Cyclopropyl oder t-Butyl steht und
sich der Substituent R¹ vorzugsweise in meta- oder para-Position, besonders bevorzugt in 4-Position (para zur NO₂-Gruppe) des Aromaten befindet;
der Formel (VI) 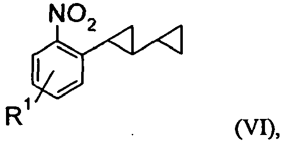 wobei
- R¹: für Halogen, -CR'(CF₃)₂ steht und R' ausgewählt ist aus H, F oder O-C₁₋₄-Alkyl und sich der Substituent R¹ vorzugsweise in meta- oder para-Position, besonders bevorzugt in 4-Position (para) des Aromaten befindet;
und der Formel (V) 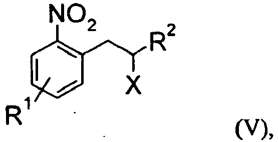 wobei
- R¹: für Wasserstoff, Halogen, -CR'(CF₃)₂ steht und R' ausgewählt ist aus H, F oder O-C₁₋₄-Alkyl) und besonders bevorzugt für Wasserstoff ist,
- R²: für Cyclopropyl oder t-Butyl steht,
- X: für ein Halogenatom, bevorzugt für Cl oder Br oder für eine Abgangsgruppe, wie Tosylat (OTos), Mesylat (OMes) oder OSOCl, steht und
sich der Substituent R¹ vorzugsweise in meta- oder para-Position, besonders bevorzugt in 4-Position (para) des Aromaten befindet.

Im Zusammenhang mit der vorliegenden Erfindung umfasst der Begriff Halogene (X) Elemente, die ausgewählt sind aus der Gruppe bestehend aus Chlor, Brom und Iod, wobei Chlor und Brom bevorzugt und Chlor besonders bevorzugt verwendet wird.

Gegebenenfalls substituierte Reste können einfach oder mehrfach substituiert sein, wobei bei Mehrfachsubstitutionen die Substituenten gleich oder verschieden sein können.

Die Definition C₁-C₄-Alkyl umfasst den größten hierin definierten Bereich für einen Alkylrest. Im Einzelnen umfasst diese Definition die Bedeutungen Methyl, Ethyl, n-, iso-Propyl, n-, iso-, sec- und t-Butyl.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Das erfindungsgemäße Verfahren kann durch folgende Schema (I) beispielhaft dargestellt werden:

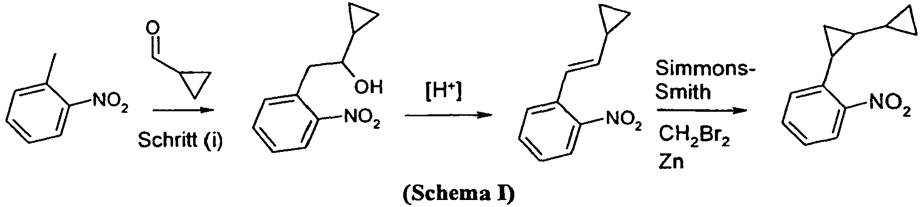

### Schritt (i)

Die erfindungsgemäße Umsetzung der 2-Nitrotoluole mit den Aldehyden gemäß Schritt (i) erfolgt vorzugsweise in Anwesenheit einer Base.

Dazu kommen z.B. Basen in Frage, die ausgewählt sind aus der Gruppe bestehend aus Alkali- und Erdalkalihydroxiden, Carbonaten und Hydriden, wobei Alkalihydroxide bevorzugt und NaOH oder KOH besonders bevorzugt verwendet werden.

Geeignete Lösungsmittel sind alle unter den vorherrschenden Reaktionsbedingungen inerten Lösungsmittel, wobei Dimethylformamid (DMF), Dimethylacetamid, N-Methylpyrrolidon (NMP), Dimethylsulfoxid (DMSO) und Dimethylenpropylharnstoff (DMPU) bevorzugt verwendet werden.

### Schritte (ii) und (iii)

In einer alternativen Ausführungsform der vorliegenden Erfindung, die durch das folgende Schema II allgemein erläutert werden soll, wird der Alkohol (IV), den aus J. of Physical ORG. Chem. 18, 2005, 9-20 bekannt ist, zunächst in ein Halogenid (V), wie z.B. ein Chlorid, überführt, welches dann zum Alken (I) eliminiert wird:

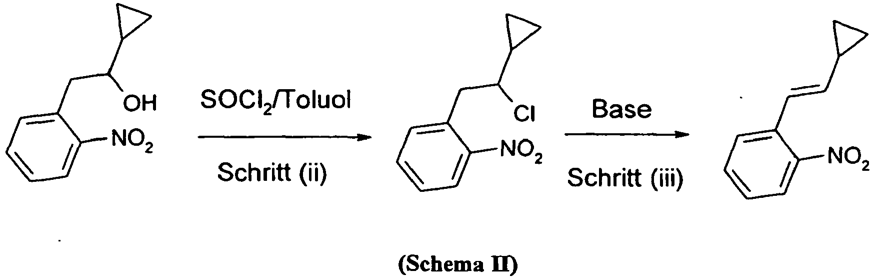

Die Halogenierung des Alkohols (IV) zum Halogenid (V) gemäß Schritt (ii) erfolgt vorzugsweise mit einem Halögenidbildner, der ausgewählt ist aus der Gruppe der Säurechloride, wie Thionylchlorid (SOCl₂), POCl₃, COCl₂ und (COCl)₂.

In einer bevorzugten Ausführungsform der Erfindung erfolgt die Eliminierung der Halogenalkylnitrobenzole der Formel (V) gemäß Schritt (iii) Basen-katalysiert oder thermisch.

Für den erfindungsgemäßen Basen-katalysierten Eliminierungsschritt werden Basen verwendet, die, z.B. ausgewählt sind aus der Gruppe bestehend aus Aminen wie Diethylamin, Dipropylamin, Diisopropylethylamin, Dibutylamin, Dicyclohexylamin, Piperidin, Triethylamin, Tripropylamin, Tributylamin, 1,8-Diazabicyclo[5.4.0]undec-7-ene (DBU), 1,4-Diazabicyclo[2.2.2]octan (DABCO); Alkoholaten wie Natriummethanolat, Kalium-t-butylat; Alkali- und Erdalkalihydroxide wie KOH, NaOH, LiOH, Ca(OH)₂; oder Carbonate wie Soda, Pottasche.

Die Basen-katalysierte Eliminierung erfolgt erfindungsgemäß bei Normaldruck und Temperaturen im Bereich von -10 bis 190 °C, vorzugsweise 0 bis 140 °C , besonders bevorzugt 10 bis 100°C.

Die Eliminierung kann in einer weiteren erfindungsgemäßen Ausführungsform auch thermisch spontan während der Umsetzung mit dem Halogenidbildner erfolgen, wie in dem nachfolgenden Schema III dargestellt ist:

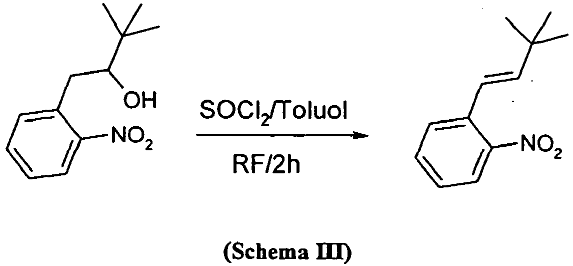

### Schritt (iv)

In einer alternativen Ausführungsform der Erfindung erfolgt die direkte Eliminierung der Hydroxyalkylnitrobenzole der Formel (IV) gemäß Schritt (iv) Säure-katalysiert oder thermisch.

Für den erfindungsgemäßen Säure-katalysierten Eliminierungsschritt werden Säuren verwendet, die ausgewählt sind aus der Gruppe bestehend aus Mineralsäuren wie Salzsäure, Schwefelsäure, Phosphorsäure, sauren Ionenaustauschern, organischen Säuren wie Methansulfonsäure, Benzolsulfonsäure, p-Toluolsulfonsäure (PTSA), Trifluoressigsäure und Essigsäure, wobei p-Toluolsulfonsäure und Schwefelsäure besonders bevorzugt verwendet werden.

Die Säure-katalysierte Eliminierung erfolgt erfindungsgemäß bei Normaldruck und Temperaturen im Bereich von 0 bis 200 °C, vorzugsweise von 20 bis 150 °C ..

Sowohl die Säure-katalysierte, als auch die thermische Eliminierungsreaktion erfolgen vorzugsweise unter gleichzeitigem Abdestillieren des bei der Reaktion freiwerdenden Wassers.

Alternativ kann die Reaktion in Gegenwart eines wasserbindenden Mittels, wie z.B. einem Säureanhydrid, wie Acetanhydrid, durchgeführt werden.

Geeignete Lösungsmittel für die Wasserabspaltung sind z.B. ausgewählt aus der Gruppe bestehend aus Aromaten wie Toluol, Xylol, Benzol, Chlorbenzol, Dichlorbenzol; Aliphaten wie Hexan, Heptan, Cyclohexan, Methylcyclohexan und Petrolether.

Die thermische Eliminierung erfolgt erfindungsgemäß bei Normaldruck und Temperaturen im Bereich von 40 bis 200 °C, vorzugsweise 50 bis 150 °C.

Die so erhaltenen 2-Alkenyl-nitrobenzole gemäß der Formel (I) sind wertvolle Zwischenprodukte für die Herstellung von Agrowirkstoffen und können durch Reduktion in 2-Alkylaniline übergeführt werden.

Alternativ können sie auch durch Cyclopropanierung in die entsprechenden Cyclopropylnitrobenzole überführt werden.

Erfindungsgemäß erfolgt die Cyclopropanierung durch Simmons-Smith-Reaktion mit Dihalogenmethan und Zink und/oder Kupfer oder Diethylzink. Die Reaktionsbedingungen der Cyclopropanierung sind dem Fachmann bekannt und im Stand der Technik, z.B. in Org. React. 1973, 20, S. 1-131 vorbeschrieben.

Alternativ kann die Cyclopropanierung auch durch Carbenaddition mit Diazomethan erfolgen.

In einer weiteren alternativen erfindungsgemäßen Ausführungsform, die durch das folgende Schema (IV) erläutert werden soll, wird zunächst die Nitrogruppe der 2-Hydroxyalkylnitrobenzole zum Amin reduziert .

Die so erhaltenen 2-Hydroxyalkylaniline lassen sich auf vergleichbare Weise eliminieren und hydrieren.

Die Reaktionsbedingungen der Hydrierung sind dem Fachmann bekannt und im Stand der Technik, z. B. in Becker, H.G.D. et al, Organikum (1976), Interdruck, Leipzig vorbeschrieben. Besonders bevorzugt erfolgt die Hydrierung in der Flüssig- und/oder Gasphase in Anwesenheit von geeigneten Hydrierkatalysatoren. Als Katalysatoren eigenen sich insbesondere Pd/C, PtO₂ und Raney-Nickel.

Die Hydrierung wird üblicherweise mit Wasserstoffdrucken von 1 bis 100 bar, vorzugsweise 2 bis 30 bar, besonders bevorzugt 5 bis 10 bar und bei Temperaturen im Bereich von 0 bis 150 °C, vorzugsweise 10 bis 100 °C und besonders bevorzugt 15 bis 50 °C durchgeführt.

Alternativ kann die Hydrierung mit Hydrierungsreagenzien, die ausgewählt sind aus Zn, Fe, SnCl₂, Sn und Dithionit erfolgen.

Die Hydrierung kann in Gegenwart einer Säure erfolgen. Als Wasserstoffquellen kommen auch Formiate und Hydrazin in Frage.

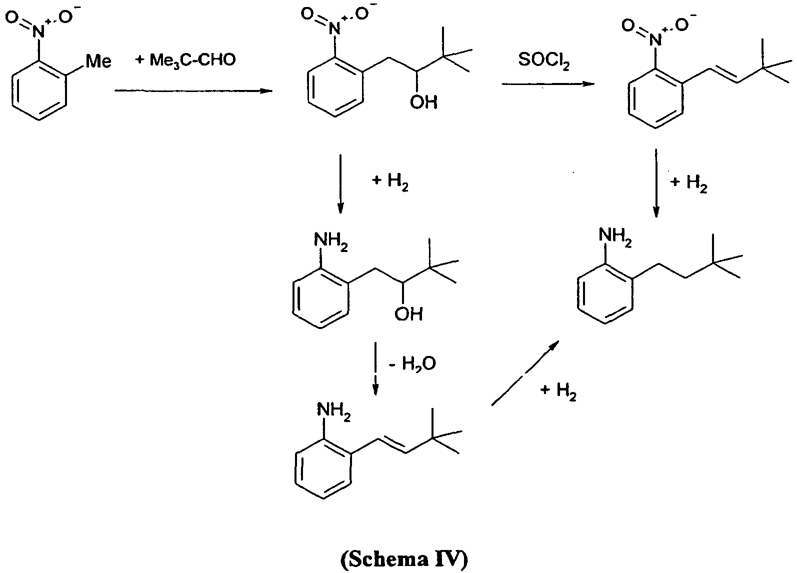

Die folgenden Beispiele sollen den Gegenstand der vorliegenden Erfindung näher erläutern, ohne ihn jedoch auf diese zu beschränken.

### Ausführungsbeispiele

### 1-Cyclopropyl-2-(2-nitrophenyl)ethanol

Zu 4,9 g (35,7 mmol) 2-Nitrotoluol in 30 ml DMF werden innerhalb einer Stunde 0,168 g (2,55 mmol; Reinheit 85%) KOH und simultan 5 g (71,34 mmol) Cyclopropylcarboxaldehyd zugegeben. Es wird 0,5 h bei Raumtemperatur (RT) gerührt, worauf 0,5 Äquivalente Cyclopropylcarboxaldehyd zugesetzt und eine weitere Stunde bei RT nachgerührt wird. Anschließend wird im Vakuum einen Grossteil des DMF abdestilliert, der Rückstand mit Wasser versetzt und mit Essigsäureethylester extrahiert. Nach dem Verdampfen des Lösungsmittels und Chromatögraphie über Kieselgel (Elution mit Toulol/Essigester-Gemischen) werden 4,2 g (Ausb. 55 % d. Th., Reinheit (GCMS 95,9%) 1-Cyclopropyl-2-(2-nitrophenyl)ethanol in Form eines Öls erhalten.

¹H-NMR. (CDCl₃): 0,25-0,33 (2 m, 2H), 0,51-0,55 (2 m, 2H), 0,94-0,99 (m, 1H), 1,85 (broad s, 1H), 3,08-3,21 (2m, 2H), 3,36 (dd, 1H), 7,36 (t, 1H), 7,45 (d, 1H), 7,53 (t, 1H), 7,9 (d, 1H).

### 1-(2-Chloro-2-cyclopropylethyl)-2-nitrobenzol

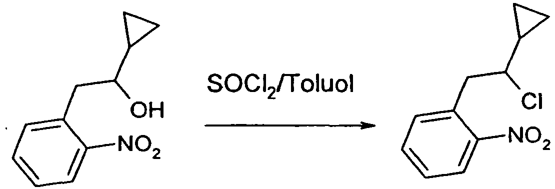

Zu 4 g (18,5 mmol; Reinheit 96%) 1-Cyclopropyl-2-(2-nitrophenyl)ethanol in 50 ml Toluol werden bei Raumtemperatur 2,3 g (19,5 mmol) Thionylchlorid zugegeben und anschließend bei 80°C 0,5 h nachgerührt. Dann werden nochmals 460 mg Thionylchlorid hinzugegeben und weitere 45 Minuten bei Raumtemperatur nachgerührt.

Nach Eindampfen im Vakuum, Lösen in Essigsäureethylester und Waschen mit gesättigter Natriumbicarbonatlösung werden 3,5 g (80 % d. Th.) 1-(2-chloro-2-cyclopropylethyl)-2-nitrobenzol erhalten.

¹H-NMR (400 MHz, d⁶-DMSO): 0,32-0,38 (2 m, 2H), 0.87-0.92 (m, 2H), 0,58-0,63 (2 m, 2H), 1,23-1,26 (m, 1H), 3,38 (dd, 1H), 3,58 (dd, 1H), 3,6-3,7 (m, 1H), 7,54 (t, 1H), 7,55 (d, 1H), 7,65 (t, 1H), 7,98 (d, 1H).

### Umsetzung von 2-Nitrotoluol mit Pivalaldehyd

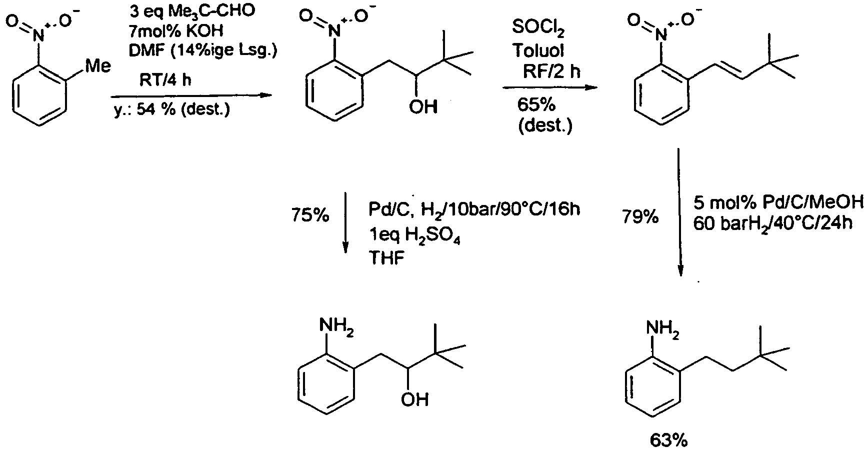

### 3,3-Dimethyl-1-(2-nitrophenyl)butan-2-oi

Zu 153,9 g (1,12 mol) 2-Nitrotoluol in 900 ml DMF werden innerhalb einer Stunde 4,5 g (80.2 mmol) KOH und simultan 193,3 g (2,24 mol) Pivalaldehyd zugegeben. Anschließend werden zunächst weitere 0,5 Äquivalente Pivalaldehyd zugefügt und eine Stunde bei RT nachgerührt, worauf nochmals 0,5 Äquivalente Pivalaldehyd hinzugefügt und weitere 2 Stunden bei Raumtemperatur nachgerührt werden. Die Mischung wird mit 40 ml 10-proz. wässriger Salzsäure bis zum Farbumschlag (pH4,5) angesäuert und ein Großteil des DMF im Vakuum abdestilliert. Nach Destillation des Rückstands bei 130 °C/0,9 mbar werden 136,8 g (Ausb. 54 % d. Th., Reinheit (GCMS 98 %) 3,3-Dimethyl-1-(2-nitrophenyl)butan-2-ol erhalten.

¹H-NMR (CDCl₃): 1,01 (s, 9H), 1,8 (broad s, 1H), 2,81 (dd, 1H), 3,22 (d, 1H), 3,48 (d, 1H), 7,37 (t, 1H), 7,47 (d, 1H), 7,53 (t, 1H), 7,89 (d, 1H).

### 1-[3,3-Dimethylbut-1-en-1-yl]-2-nitrobenzol

Zu 40 g (179 mmol) 3,3-Dimethyl-1-(2-nitrophenyl)butan-2-ol werden in 400 ml Toluol bei Raumtemperatur 23,44 g (197 mmol) Thionylchlorid zugegeben und 2 h unter Rückfluss gekocht. Nach Waschen mit 100 ml gesättigter Natriumbicarbonatlösung und Destillation der organischen Phase bei 116°C/12 mbar werden 20 g (48 % d. Th.) 1-[(1E)-3,3-Dimethylbut-1-en-1-yl]-2-nitrobenzol erhalten.

¹H-NMR (CDCl₃): 1,14 (s, 9H), 6,23 (d, 1H), 6,78 (d, 1H), 7,34 (t, 1H), 7,52 (t, 1H), 7,57 (d, 1H), 7,88 (d, 1H).

### 1-(2-Aminophenyl)-3,3-dimethylbutan-2-ol

3 g (13,4 mmol) 3,3-Dimethyl-1-(2-nitrophenyl)butan-2-ol werden in 50 ml THF mit 2,06 g 65-proz. Schwefelsäure und 0,214 g Palladium/Kohle (10%ig) bei 90°C unter 10 bar Wasserstoffdruck 16 Stunden lang gerührt. Anschließend wird die Lösung über Celite abgesaugt und das Filtrat einrotiert. Nach Lösen in Essigsäureethylester, Waschen mit gesättigter Natriumbicarbonatlösung, Abtrennen und Eindampfen der organischen Phase im Vakuum erhält man 2,2 g (75% d. Th.) eines Feststoffs. Reinheit (GC/MS): 88 %.

MS (m/e): 193, 160, 136, 107 (100%).

### 2-(3,3-Dimethylbutyl)anilin

1 g (4,87 mmol) 1-[3,3-Dimethylbut-1-en-1-yl]-2-nitrobenzol werden in 20 ml Methanol mit 0,26 g Palladium/Kohle (10%ig) bei 40°C unter 60 bar Wasserstoffdruck 24 Stunden lang gerührt. Anschließend wird die Lösung über Celite abgesaugt und das Filtrat einrotiert. Man erhält 0,8 g (58% d. Th.) eines Öls. Reinheit (GC/MS): 63 %.

¹H-NMR (CDCl₃): 0,99 (s, 9H), 1,5 (m, 2H), 2,45 (m, 2H), 3,6 (broad s, 2H), 6,67 (d, 1H), 6,68 (t, 1H), 7,01-7,05 (m, 2H).

### 1-[2-Cyclopropylvinyl]-2-nitrobenzol

Zu 1 g (4,25 mmol, Reinheit 96%) 1-(2-Chloro-2-cyclopropylethyl)-2-nitrobenzol in 20 ml Dioxan werden 0,65 g (4,25 mmol) 1,8-Diazabicyclo(5.4.0)undec-7-en gegeben und 12 h bei Raumtemperatur gerührt. Anschließend wird das Lösungsmittel im Vakuum eingedampft und der Rückstand mit Ethylacetat und Wasser versetzt. Nach Abtrennen der Organischen Phasen und Eindampfen des Lösungsmittels werden 0,7 g (79 % d. Th.) 1-[2-Cyclopropylvinyl]-2-nitrobenzol erhalten.

¹H-NMR (400 MHz, CDCl₃): 0,55-0,59 (m, 2H), 0,87-0,92 (m, 2H), 1,61-1,69 (m, 2H), 5,75 (dd, 1H), 6,94 (d, 1H), 7,3 (m, 1H), 7,47-7,57 (m, 2H), 7,86 (d, 1H).

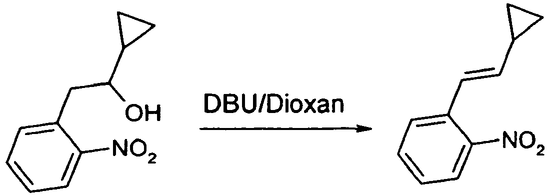

## Patentansprüche

1. Alkenylnitwbenzole der Formel (I) 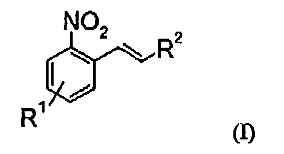 wobei
R¹ für Halogen, -CR'(CF₃)₂ steht und R' ausgewählt ist aus =H, F oder O-C₁₋₄-Alkyl und
R² für t-Butyl oder Cyclopropyl steht.

2. Alkenylnitrobenzole der Formel (VI) 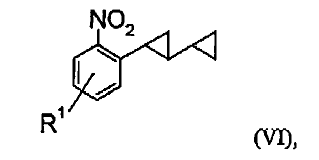 wobei
R¹ für Halogen, -CR'(CF₃)₂ steht und R' ausgewählt ist aus H, F oder O-C₁₋₄-Alkyl.

3. Alkenylnitrobenzole der Formel (V) 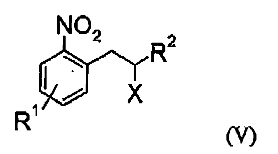 wobei
R¹ für Wasserstoff, Halogen, -CR'(CF₃)₂ steht und R' ausgewählt ist aus H, F oder O-C₁₋₄-Alkyl,
X für ein Halogenatom oder eine Abgangsgruppe steht und
R² für Cyclopropyl oder t-Butyl steht.

4. Verfahren zum Herstellen von Alkenylnitrobenzolen der Formel (I) 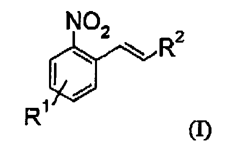 wobei
R¹ für Wasserstoff, Halogen, -CR'(CF₃)₂ steht und R' ausgewählt ist aus H, F oder O-C₁₋₄-Alkyl und
R² für Cyclopropyl oder t-Butyl steht,
umfassend die Schritte
(i) Umsetzen von 2-Nitrotoluolen der Formel (II), 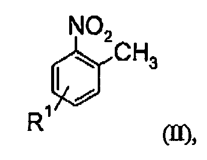 worin
R¹ die obige Bedeutung hat, mit Aldehyden der Formel (III),
R²-CHO (III),
worin
R² die obige Bedeutung hat,
in Gegenwart einer Base zu Hydroxalkylnitrobenzolen der Formel (IV);
(ii) Umsetzen und der aus Schritt (i) erhaltenen Hydroxyalkylnitrobenzole der Formel (IV) 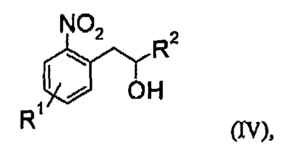
worin R¹ und R² die obigen Bedeutungen haben, zu Halogenaikylnitrobenzolen der Formel (V) 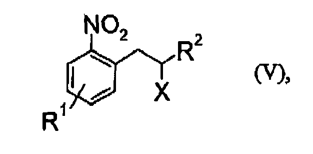
worin R¹ und R² die obigen Bedeutungen haben und X ein Halogenatom oder eine Abgangsgruppe ist,
und
(iii) anschließende Eliminierung der Halogenalkylnitrobenzole der Formel (V) zu Alkenylnitrobenzolen der Formel (I)
oder
(iv) direkte Eliminierung der Hydroxyalkylnitrobenzole der Formel (IV) zu Alkenylnitrobenzolen der Formel (I).

5. Verfahren gemäß Anspruch 4, **dadurch gekennzeichnet, dass** die Eliminierung der Halogenalkylnitrobenzole der Formel (V) gemäß Schritt (iii) Basen-katalysiert oder thermisch erfolgt.

6. Verfahren gemäß einem der Ansprüche 4 oder 5, **dadurch gekennzeichnet, dass** die direkte Eliminierung der Hydroxyalkylnitrobenzole der Formel (IV) gemäß Schritt (iv) Säure-katalysiert oder thermisch erfolgt,

7. Verfahren gemäß einem der Ansprüche 4 bis 6, **dadurch gekennzeichnet, dass** vor der Eliminierung, gemäß Schritten (iii) oder (iv), zunächst die Nitrogruppe der Hydroxyalkylnitrobenzole zum Amin reduziert wird.

## Claims

1. Alkenylnitrobenzenes of the formula (I) 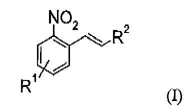 where
R¹ is halogen, -CR'(CF₃)₂ and R' is selected from H, F and O-C₁₋₄-alkyl and
R² is t-butyl or cyclopropyl.

2. Alkenylnitrobenzenes of the formula (VI) 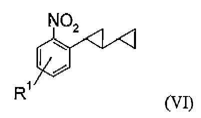 where
R¹ is halogen, -CR'(CF₃)₂ and R' is selected from H, F and O-C₁₋₄-alkyl.

3. Alkenylnitrobenzenes of the formula (V) 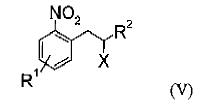 where
R¹ is hydrogen, halogen, -CR'(CF₃)₂ and R' is selected from H, F and O-C₁₋₄-alkyl,
X is a halogen atom or a leaving group and
R² is cyclopropyl or t-butyl.

4. Process for preparing alkenylnitrobenzenes of the formula (I) 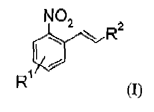 where
R¹ is hydrogen, halogen, -CR'(CF₃)₂ and R' is selected from H, F and O-C₁₋₄-alkyl and
R² is cyclopropyl or t-butyl,
comprising the steps of
(i) reacting 2-nitrotoluenes of the formula (II) 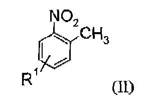 in which
R¹ is as defined above with aldehydes of the formula (III),
R²-CHO (III)
in which
R² is as defined above
in the presence of a base to give hydroxyalkylnitrobenzenes of the formula (IV);
(ii) converting the hydroxyalkylnitrobenzenes of the formula (IV) obtained from step (i) 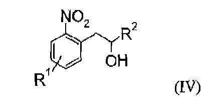
in which R¹ and R² are each as defined above to haloalkylnitrobenzenes of the formula (V) 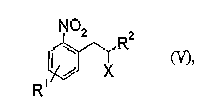
in which R¹ and R² are each as defined above and X is a halogen atom or a leaving group,
and
(iii) subsequently eliminating the haloalkylnitrobenzenes of the formula (V) to give alkenylnitrobenzenes of the formula (I)
or
(iv) directly eliminating the hydroxyalkylnitrobenzenes of the formula (IV) to give alkenylnitrobenzenes of the formula (I).

5. Process according to Claim 4, **characterized in that** the haloalkylnitrobenzenes of the formula (V) are eliminated in step (iii) under base catalysis or thermally.

6. Process according to either of Claims 4 and 5, **characterized in that** the hydroxyalkylnitrobenzenes of the formula (IV) are eliminated directly in step (iv) under acid catalysis or thermally.

7. Process according to any one of Claims 4 to 6, **characterized in that** the elimination in step (iii) or (iv) is preceded by preliminary reduction of the nitro group of the hydroxyalkylnitrobenzenes to the amine.

## Revendications

1. Alcénylnitrobenzènes de formule (I) 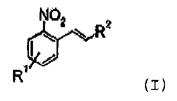 dans laquelle
R¹ représente un atome d'halogène, -CR' (CF₃)₂ et R' est choisi parmi H, F ou un groupe O-alkyle(C₁-C₄) et
R² représente le groupe tert-butyle ou cyclopropyle.

2. Alcénylnitrobenzènes de formule (VI) 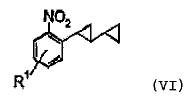 dans laquelle
R¹ représente un atome d'halogène, -CR'(CF₃)₂ et R' est choisi parmi H, F ou un groupe O-alkyle(C₁-C₄).

3. Alcénylnitrobenzènes de formule (V) 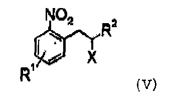 dans laquelle
R¹ représente un atome d'hydrogène ou d'halogène, -CR' (CF₃)₂ et R' est choisi parmi H, F ou un groupe O-alkyle(C₁-C₄),
X représente un atome d'halogène ou un groupe partant et
R² représente le groupe cyclopropyle ou tert-butyle.

4. Procédé pour la préparation d'alcénylnitrobenzènes de formule (1) 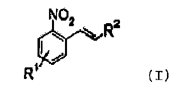 dans laquelle
R¹ représente un atome d'hydrogène ou d'halogène, -CR' (CF₃)₂ et R' est choisi parmi H, F ou un groupe O-alkyle(C₁-C₄) et
R² représente le groupe cyclopropyle ou tert-butyle,
comprenant les étapes
(i) mise en réaction de 2-nitrotoluènes de formule (II), 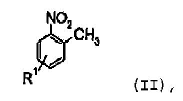 dans laquelle
R¹ a la signification ci-dessus,
avec des aldéhydes de formule (III)
R²-CHO (III),
dans laquelle
R² a la signification ci-dessus,
en présence d'une base, pour l'obtention d'hydroxyalkylnitrobenzènes de formule (IV) ;
(ii) mise en réaction des hydroxyalkylnitrobenzènes obtenus dans l'étape (i), de formule (IV) 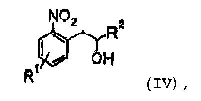
dans laquelle R¹ et R² ont les significations ci-dessus, pour l'obtention d'halogénoalkyl-nitrobenzènes de formule (V), 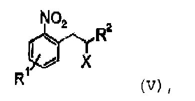
dans laquelle R¹ et R² ont les significations ci-dessus et X est un atome d'halogène ou un groupe partant,
et
(iii) réaction d'élimination subséquente dans les halogénoalkylnitrobenzènes de formule (V) pour l'obtention d'alcénylnitrobenzènes de formule (I)
ou
(iv) réaction d'élimination directe dans les hydroxyalkylnitrobenzènes de formule (IV) pour l'obtention d'alcénylnitrobenzènes de formule (I).

5. Procédé selon la revendication 4, **caractérisé en ce que** la réaction d'élimination dans les halogénoalkylnitrobenzènes de formule (V) selon l'étape (iii) s'effectue thermiquement ou avec catalyse par des bases.

6. Procédé selon la revendication 4 ou 5, **caractérisé en ce que** la réaction d'élimination dans les hydroxyalkylnitrobenzènes de formule (IV) selon l'étape (iv) s'effectue thermiquement ou avec catalyse par des acides.

7. Procédé selon l'une quelconque des revendications 4 à 6, **caractérisé en ce qu'**avant la réaction d'élimination selon l'étape (iii) ou (iv) d'abord on réduit en le groupe amino le groupe nitro des hydroxyalkylnitrobenzènes.
